# EUROPEAN PATENT APPLICATION

(11) **EP 4 160 605 A1**
(43) Date of publication of application: **05.04.2023**
(21) Application number: 21817105.6
(22) Date of filing: 21.04.2021
(51) Int. Cl.: G16H 10/40, G16H 20/60

(54) **NUTRITION STATE ANALYSIS SYSTEM USING URINE TEST STRIP**

(30) Priority: 02.06.2020 JP 2020095880
(71) Applicant: Yukashikado Inc., Tokyo 150-0001 (JP)
(72) Inventor: MINOBE, Shinya, Tokyo 150-0001 (JP); SAITO, Keiichiro, Tokyo 150-0001 (JP)
(74) Representative: Fleck, Hermann-Josef
(86) International application number: PCT/JP2021/016198
(87) International publication number: WO 2021/246074

(57) **Abstract**

A nutrition state analysis system in which a service provision server and a client terminal transmit and receive data via a network, wherein: the client terminal is provided with a urine test strip color data input means, and a data transmission/reception means that transmits color data for an inputted urine test strip to the service provision server and receives evaluation data from the service provision server; and the service provision server is provided with a urine test strip color data analysis means, a nutrition state evaluation means that evaluates the nutrition state of the user on the basis of the analysis result produced by the urine test strip color data analysis means, and a server-side data transmission/reception means that receives the color data for the urine test strip from the client terminal, and transmits evaluation data from the nutrition state evaluation means to the client terminal.

## Description

### [Technical Field]

The present invention relates to a technique for analyzing a user's nutrition state using urine test strips.

### [Background Art]

In recent years, the number of people who take health foods and supplements is increasing due to heightened awareness of health. It is important for users to accurately grasp their own nutrition state in order to select health foods and the like that are suitable for them. From body fluids such as blood and urine, it is possible to grasp the condition of one's own body, that is, the presence or absence of disease and the nutrition state of the body. However, in order for users to know their own nutrition state, it is necessary to use special and expensive testing equipment for blood and urine tests, and it was not something that anyone could easily use, such as being tested at a hospital.

As a urine test system which anyone could easily use, a urine test system using spot urine has already been proposed (see Patent Document 1). This urine test system stabilizes urinary vitamins for several days, making a urine test convenient for the user. The urine test system uses a system in which a client terminal on the user side and a server on the service provider side are connected via a network to transmit and receive data. The client terminal transmits the user information and test application information which the users input as data. The server assigns the user identification information based on the received data, outputs the urine collection kit delivery instruction data, inputs the analysis results of the urine components collected by the user using the urine collection kit, evaluates and judges the urine components, and outputs the result data of the evaluation judgment. The urine collection kit contains a stabilizing agent or stabilizing reagent that stabilizes vitamins in urine for at least 3 days.

However, in such a urine test system, since urine is exchanged by mail using the urine collection kit, it takes several days to obtain the test results, and the user cannot immediately grasp the nutrition state on the day of urine collection.

### [Prior Art]

### [Patent Document]

[Patent Document 1] WO 2018/047883

### [Outline of the Invention]

### [Problems to be Solved by the Invention]

In view of such circumstances, an object of the present invention is to provide a nutrition state analysis system with which a user can easily and quickly learn his or her own nutrition state.

### [Means to Solve the Objects]

In order to solve the above-mentioned problems, a nutritional state analysis system of a first aspect of the present invention is a system in which a client terminal and a service provision server transmit and receive data via a network, and each has the following means.

The client terminal is provided with 1A) a urine test strip color data input means for inputting the color data of the urine test strip soaked with collected urine, and 1B) a data transmission/reception means that transmits color data for an inputted urine test strip to the service provision server and receives evaluation data from the service provision server.

Also, the service provision server is provided with 1a) a urine test strip color data analysis means for analyzing color data of urine test strip; 1b) a nutrition state evaluation means that evaluates the nutrition state of the user on the basis of the analysis result produced by the urine test strip color data analysis means; and 1c) a server-side data transmission/reception means that receives the color data for the urine test strip from the client terminal, and transmits evaluation data from the nutrition state evaluation means to the client terminal.

According to the system of the present invention, the urine collected by the user can be tested by the user using the urine test strip, and the mineral content in the urine can be discriminated by color. Also, by sending the color information to the service provider side via the network, the nutrition state of the user can be quickly grasped.

This allows the user to easily use the nutrition state analysis system.

A nutritional state analysis system of a second aspect of the present invention is a system in which a client terminal and a service provision server transmit and receive data via a network, and each has the following means.

The client terminal is provided with 2A) a urine test strip color data input means for inputting the color data of the urine test strip soaked with collected urine; 2B) a question data input means for inputting question data regarding physical condition on the day urine was collected; and 2C) a data transmission/reception means that transmits color data for an inputted urine test strip and question data to the service provision server and receives evaluation data from the service provision server.

The service provision server is provided with 2a) a urine test strip color data analysis means for analyzing color data of the urine test strip; 2b) a question data analysis means for analyzing question data; 2c) a user information memory means for storing user information; 2d) a nutrition state evaluation means that evaluates the nutrition state of the user on the basis of the analysis result respectively analyzed by the urine test strip color data analysis means and the question data analysis means and the user information stored in the user information memory means; and 2e) a server-side data transmission/reception means that receives the color data for the urine test strip and the question data from the client terminal, and transmits evaluation data from the nutrition state evaluation means to the client terminal.

In the nutrition state analysis system of the second aspect of the present invention, as compared with the system of the first aspect described above, the client terminal further comprises the question data input means regarding the physical condition on the day urine was collected, and the service provision server further comprises the question data analysis means, whereby it becomes possible to evaluate the nutrition state in consideration of changes in physical condition and symptoms recognized by the user himself/herself on the day of urine collection.

The user information stored in the user information storage means includes basic information such as gender and age, and questionnaire information such as lifestyle habits and medical history, and includes a database that contains the user's nutrition state already obtained outside of urine test strip testing. With such information, it is possible to grasp the eating habits of the user. Information on the user and other users is stored in the database.

A nutritional state analysis system of a third aspect of the present invention is a system in which a client terminal and a service provision server transmit and receive data via a network, and each has the following means.

The client terminal is provided with 3A) a urine test strip color data input means for inputting the color data of the urine test strip soaked with collected urine; 3B) a meal data input means for entering meal data on the day urine was collected; 3C) a question data input means for inputting question data regarding physical condition on the day urine was collected; and 3D) a data transmission/reception means that transmits color data for an inputted urine test strip, meal data and question data to the service provision server and receives evaluation data from the service provision server.

The service provision server is provided with 3a) a urine test strip color data analysis means for analyzing color data of the urine test strip; 3b) a meal data analysis means for analyzing meal data; 3c) a question data analysis means for analyzing question data; 3d) a user information memory means for storing user information; 3e) a nutrition state evaluation means that evaluates the nutrition state of the user on the basis of the analysis result respectively analyzed by the urine test strip color data analysis means, the meal data analysis means and the question data analysis means, and the user information stored in the user information memory means; and 3f) a server-side data transmission/reception means that receives the color data for the urine test strip, the meal data and the question data from the client terminal, and transmits evaluation data from the nutrition state evaluation means to the client terminal.

In the nutrition state analysis system of the third aspect of the present invention, as compared to the system of the first or second aspect described above, the client terminal further comprises meal data input means for inputting meal data on the day urine was collected, and the service provision server further comprises meal data analysis means, whereby it becomes possible to quickly grasp the user's nutrition state together with the information on the diet the user is taking.

Here, the meal data preferably includes all of "breakfast", "lunch", "dinner" and "snack", but for example, only "breakfast" may be entered. Entering meal data on the day urine was collected does not mean that the order of urine collection and meal data is limited, and urine may be collected after entering meal data.

In the nutrition state analysis system of the first to third aspects of the present invention, the urine test strip reacts with at least one of urinary calcium ions and urinary magnesium ions to develop a color, and the color density varies depending on the ion concentration. Therefore, the urine test strip color data analysis means preferably analyzes the calcium or magnesium intake from the color data.

As a result, for example, based on a correlation with the intake data of other nutrients (sodium, etc.) that correlates with the amount of calcium excretion obtained from urine, it is possible to estimate the intake of other nutrients that are associated with calcium excretion.

In the nutrition state analysis system of the first to third aspects of the present invention, the urine test strip color data input means photographs the reaction site of the urine test strip colored by the collected urine and a reference color sample at the same time by a camera mounted on the client terminal. Then, the urine test strip color data analysis means analyzes a color code of the reaction site based on the color sample. By photographing the reaction site of the urine test strip at the same time as the color sample and using the color sample, it is possible to correct fluctuations in the photographed color due to the photographing environment such as illumination light and camera settings.

Also, in the nutrition state analysis system of the first to third aspects of the present invention, the urine test strip color data input means may acquire color information indicated by a color sample that approximates the color of the urine test strip colored by the collected urine. For example, the camera means mounted on the client terminal urges the user to photograph the color shown in the color sample and an attached color number (including the color code), and transmits the photographed image to the service provision server. And the color number (including the color code) can be analyzed by the urine test strip color data analysis means.

Further, as the urine test strip color data input means, a two-dimensional code may be displayed on the urine test strip, and the two-dimensional code may be read by the camera means mounted on the client terminal and transmitted to the service provision server.

In the nutrition state analysis system according to the third aspect of the present invention, it is preferable that the meal data input means discriminates whether or not there is an entry of meal data for the day before the urine was collected in addition to the day the urine was collected. If no meal data for the previous day has been entered, the user is prompted to enter the meal data for the day before the urine was collected. By prompting the user to enter the meal data of the day before the urine collection date, more accurate analysis of the nutrition state becomes possible.

### [Effects of the Invention]

According to the nutritional state analysis system of the present invention, by mainly using urine test strip color data, and further using meal data, question data, etc. for analysis, it is possible to analyze the nutritional state with higher accuracy. In addition, since any data can be input by a simple method, anyone can easily use it, and there is an effect that the nutrition state can be grasped quickly.

### [Brief Description of the Drawings]

[Fig. 1] Fig. 1 shows a functional block diagram of the nutrition state analysis system of Example 1.
[Fig. 2] Fig. 2 shows a schematic flow diagram of the nutrition state analysis system of Example 1.
[Fig. 3] Fig. 3 shows an input flow diagram for urine test strip color data.
[Fig. 4] Fig. 4 shows a screen display image 1 for inputting urine test strip color data.
[Fig. 5] Fig. 5 shows an explanatory diagram of urine test strip and color sample paper.
[Fig. 6] Fig. 6 shows a screen display image 2 for inputting urine test strip color data.
[Fig. 7] Fig. 7 shows a functional block diagram of the nutrition state analysis system of Example 2.
[Fig. 8] Fig. 8 shows a schematic flow diagram of the nutrition state analysis system of Example 2.
[Fig. 9] Fig. 9 shows a screen display image for inputting question data.
[Fig. 10] Fig. 10 shows a functional block diagram of the nutrition state analysis system of Example 3.
[Fig. 11] Fig. 11 shows a schematic flow diagram of the nutrition state analysis system of Example 3.
[Fig. 12] Fig. 12 shows a screen display image for inputting meal data.
[Fig. 13] Fig. 13 shows a screen display image for inputting urine test strip color data in Example 4.

### [Best Mode for Carrying Out the Invention]

Examples of the present invention will be described in detail below with reference to the drawings. The present invention is not limited to the following examples and examples shown in the figures, and the present invention can be variously changed in design.

### [Example 1]

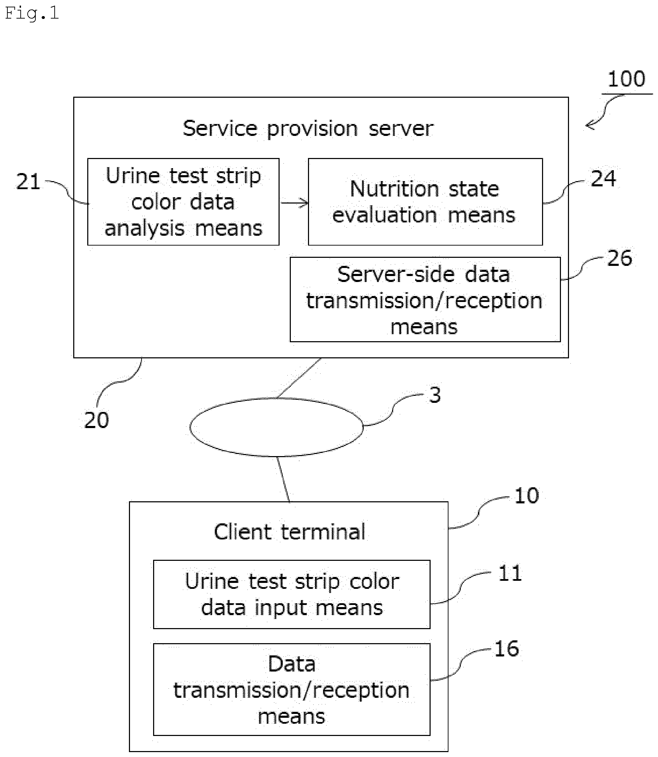

Fig. 1 shows a functional block diagram of the nutrition state analysis system of Example 1. As shown in Fig. 1, the nutrition state analysis system 100 is a system in which a service provision server 20 and a client terminal 10 transmit and receive data via the Internet 3.

The client terminal 10 is provided urine test strip color data input means 11 and data transmission/reception means 16. The urine test strip color data input means 11 inputs the color data of the urine test strip soaked in the collected urine, and the data transmission/reception means 16 transmits the input color data of the urine test strip to the service provision server 20, and receives evaluation data on nutrition state from the service provision server 20.

The service provision server 20 is provided the urine test strip color data analysis means 21, the nutrition state evaluation means 24 and server-side data transmission/reception means 26. The urine test strip color data analysis means 21 analyzes the color data of the urine test strip, and the nutrition state evaluation means 24 evaluates the nutrition state of the user (not shown) based on the analysis result analyzed by the urine test strip color data analysis means 21. Also, the server-side data transmission/reception means 26 receives the color data of the urine test strip from the client terminal 10 and transmits the evaluation data of the nutrition state evaluation means 24 to the client terminal 10.

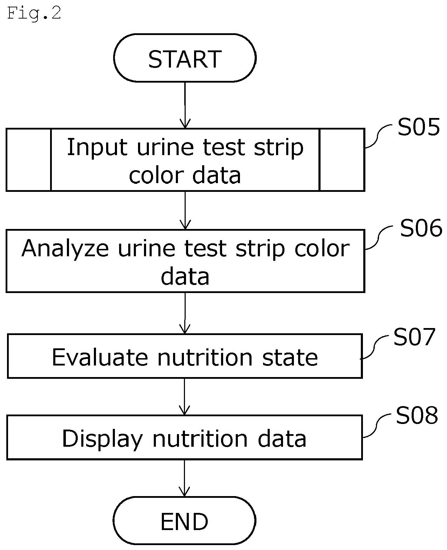

Next, a usage flow of the nutrition state analysis system of Example 1 will be described with reference to Figs. 2 to 5. Fig. 2 shows a schematic flow diagram of the nutrition state analysis system of Example 1. In addition, in each of the first to third examples, the user uses a smartphone (not shown) as the client terminal 10 to use the service.

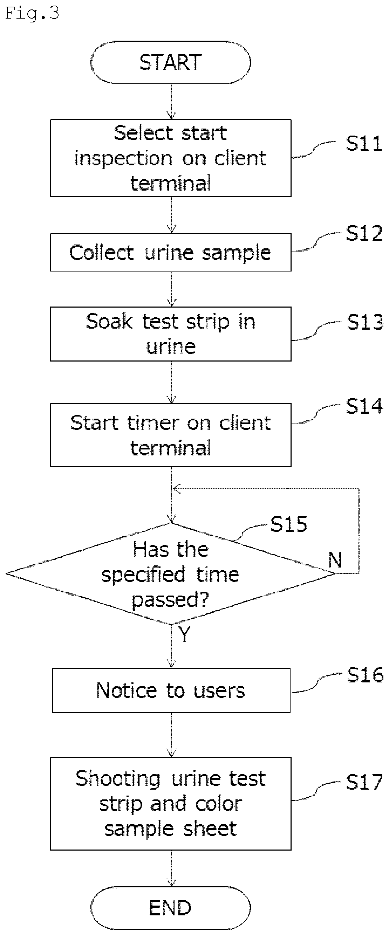

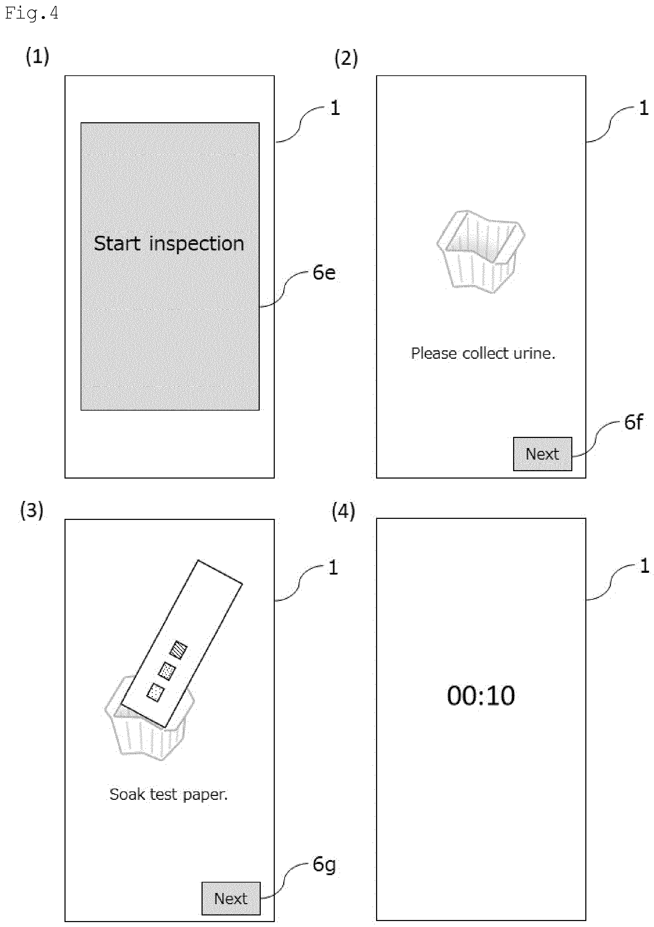

First, the user inputs urine test strip color data using the urine test strip color data input means 11 provided in the client terminal 10 (step S05). Fig. 3 shows an input flow diagram of urine test strip color data. Fig. 4 is a screen display image for inputting urine test strip color data, Fig. 4(1) is an examination start screen, Figs. 4(2) and 4(3) are procedure explanation screens, and Fig. 4(4) is a timer screen. The urine test strip used in Examples 1 to 4 includes a reactive site that reacts with urinary calcium ions to develop color and a reactive site that reacts with urinary magnesium ions to develop color. Urine test strips with different color densities depending on the ion concentration are used.

As shown in Fig. 4(1), on the examination start screen, a button 6e for prompting the examination start is displayed on the display 1. The user selects the start of inspection by tapping the button 6e (step S11).

When the examination is started, the screen transitions to the procedure explanation screen shown in Fig. 4(2). The display 1 displays an image of a urine collection container together with the message "Please collect urine". The user collects urine using the urine collection container (not shown) owned by the user (step S12). After the urine collection is completed, the user taps the button 6f labeled "Next" to switch to the procedure explanation screen shown in Fig. 4(3).

On the procedure explanation screen shown in Fig. 4(3), the display 1 displays an image of soaking the test strip into the urine collection container and the message "Please dip the test strip". The user soaks the test strip in urine according to the procedure explanation screen (step S13). After soaking the test strip in urine, when the user taps the button 6g labeled "Next", the screen transitions to the timer screen shown in Fig. 4(4), and the timer in the client terminal 10 is activated (step S14). After the timer is activated, when a predetermined time has passed (step S15), the user is notified (step S16). Here, the user is notified by a notification sound after counting for 10 seconds, but the counting time and notification method may be changed depending on the type of test strip, inspection items, and the like.

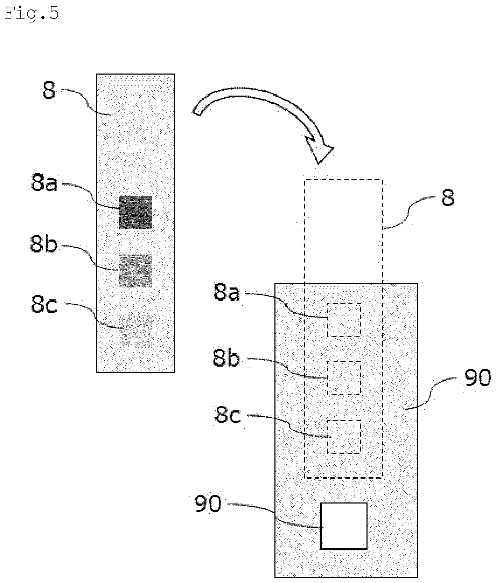

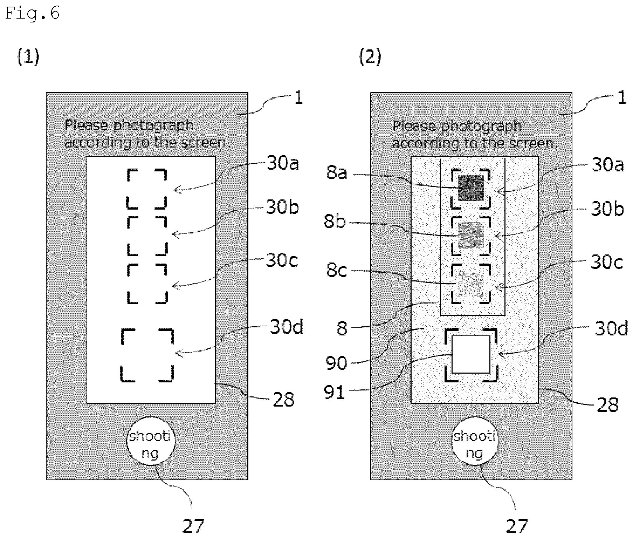

Upon receiving the notification sound, the user takes an image of the urine test strip and the color sample paper using a camera (not shown) provided on the client terminal 10 (step S17). Fig. 5 shows an explanatory diagram of a urine test strip and color sample paper. Fig. 6 is a screen display image for inputting urine test strip color data, Fig. 6(1) is shown before placement of the urine test strip and color sample paper, and Fig. 6(2) is shown after placement of the urine test strip and color sample paper.

As shown in Fig. 5, the urine test strip 8 is provided with reaction sites (8a to 8c). A color sample 90 is displayed on the color sample sheet 9. When photographing the urine test strip 8 and the color sample 90, the urine test strip 8 is overlapped on the color sample 90, and the reaction sites (8a to 8c) and the color sample 91 are placed within the imaging range.

As shown in Fig. 6(1), a shooting button 27 and an imaging range 28 are displayed on the display 1 during shooting. In addition, frame lines (30a to 30d) for positioning are displayed in the imaging range 28. The user adjusts the positions of the urine test strip 8 and the color sample sheet 90 while confirming the positions of the frame lines (30a to 30d). Specifically, as shown in Fig. 6(2), the reaction site 8a is arranged so as to be within the range of the frame line 30a. Similarly, the reaction site 8b, the reaction site 8c, and the color sample 91 are arranged so as to fall within the ranges of the frame lines 30b, 30c, and 30d, respectively. In this state, the shooting button 27 is tapped to photograph.

A shooting image photographed by tapping the shooting button 27 is transmitted to the service provision server 20 via the Internet 3 by the data transmission/reception means 16. The service provision server 20 analyzes the received urine test strip color data using the urine test strip color data analysis means 21 (step S06). Specifically, the color code of the reaction sites (8a to 8c) is analyzed using the color sample 90 as a reference.

After analyzing the urine test strip color data, the service provision server 20 evaluates the user's nutrition state based on above analysis results as shown in Fig 2. The evaluated nutrition data is transmitted to the client terminal 10 via the Internet 3 by the server-side data transmission/reception means 26 provided in the service provision server 20. The client terminal 10 displays the received evaluated nutrition data on the display 1 (step S08).

The user can view the displayed nutrition data and easily and quickly check his own nutrition state. As described above, according to the nutrition state analysis system 100, the user performs a test on the urine collected by the user himself/herself using a urine test strip, and the mineral content in the urine is discriminated by color, and the user's nutrition state can be quickly grasped by sending the color information to the service provision side via the network.

### [Example 2]

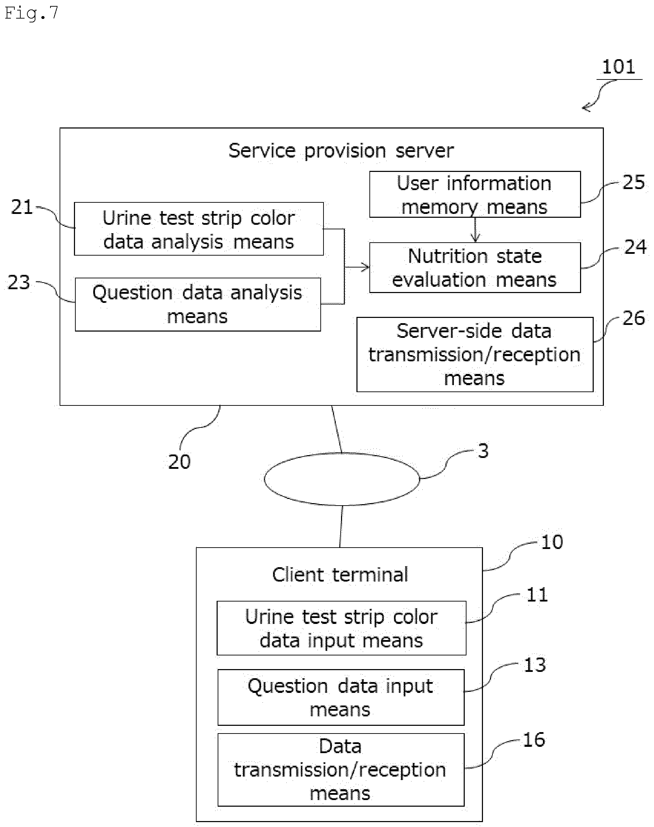

Fig. 7 shows a functional block diagram of the nutrition state analysis system of Example 2. As shown in Fig 7, the nutrition state analysis system 101 is a system in which the service provision server 20 and the client terminal 10 transmit and receive data via the Internet 3.

The client terminal 10 is provided with urine test strip color data input means 11, question data input means 13 and data transmission/reception means 16. The urine test strip color data input means 11 inputs the color data of the urine test strip soaked in the collected urine, and the question data input means 13 inputs the question data on the physical condition on the day the urine was collected. Also, the data transmission/reception means 16 transmits the input color data of the urine test strip and the question data to the service provision server 20 and receives the evaluation data regarding the nutrition state from the service provision server 20.

The service provision server 20 is provided with urine test strip color data analysis means 21, question data analysis means 23, nutrition state evaluation means 24, user information memory means 25 and server-side data transmission/reception means 26. The urine test strip color data analysis means 21 analyzes the color data of the urine test strip, and the question data analysis means 23 analyzes the question data. The user information memory means 25 stores user information. The nutrition state evaluation means 24 evaluates the user's nutrition state based on the analysis results respectively analyzed by the urine test strip color data analysis means 21 and the question data analysis means 23 and the user information stored in the user information memory means 25. The server-side data transmission/reception means 26 receives the urine test strip color data and the question data from the client terminal 10 and transmits the evaluation data of the nutrition state evaluation means 24 to the client terminal 10.

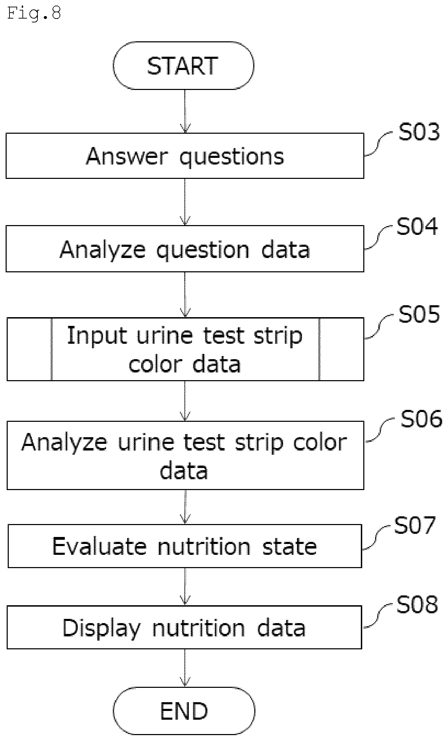

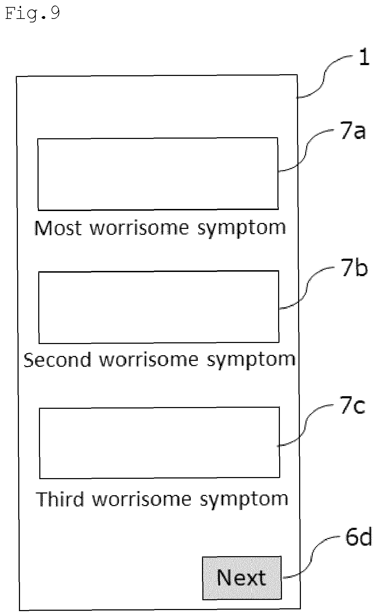

Next, a usage flow of the nutrition state analysis system of Example 2 will be described with reference to Figs. 8 and 9. Fig. 8 shows a schematic flow diagram of the nutrition state analysis system of Example 2.

As shown in Fig. 8, first, the user uses the data input means 13 provided in the client terminal 10 to answer questions (step S03). Fig. 9 shows a screen display image for inputting question data. As shown in Fig. 9, the display 1 displays symptom selection fields (7a to 7c) and a button 6d. Specifically, the symptom selection field 7a for inputting the "most worrisome symptom", the symptom selection field 7b for inputting the "second worrisome symptom", and the symptom selection field 7c for inputting the "third worrisome symptom" are displayed. The user selects each symptom selection field (7a to 7c), and selects an item suitable for him/herself from among the displayed items (not shown).

Here, only three symptom selection fields are displayed, but four or more or two or less may be displayed. In addition, the user does not necessarily have to answer all of the provided symptom selection fields, and may, for example, fill in only the symptom selection field 7a.

It should be noted that the question items in step S03 are not limited to "worrisome symptoms", and for example, question items regarding the user's weight, blood pressure, pulse rate, etc. may be provided. Further, the input method is not limited to the method of selecting from items prepared in advance, and may be, for example, a method of answering in free text.

When the user taps the button 6d labeled "Next" after selecting items suitable for him/herself for each symptom selection fields (7a to 7c), the question data is sent to the service provision server 20 via the Internet 3 by the data transmission/reception means 16. The service provision server 20 analyzes the received question data using the question data analysis means 23 (step S04).

After completing the input of the question data, the user inputs urine test strip color data using the urine test strip color data input means 11 provided in the client terminal 10 (step S05). The service provision server 20 analyzes the received urine test strip color data using the urine test strip color data analysis means 21 (step S06). The step S05 of inputting the urine test strip color data and the step S06 of analyzing the urine test strip color data are the same as in Example 1.

After analyzing the question data and urine test strip color data, the service provision server 20 evaluates (step S07) the user's nutrition state based on each analysis result and the user information stored by the user information memory means 25, using the nutrition state evaluation means 24. The user information stored in the user information memory means 25 includes not only the information of the user him/herself but also the information of other users who use the service. Also, the user information includes not only basic information such as gender and age, and questionnaire information such as lifestyle habits and medical history, but also analysis results of past meal data, question data, and urine test strip color data. Therefore, for example, it is possible to use the information of a user who has a closer analysis result to the urine test strip analysis result for the user's nutrition evaluation.

The evaluated nutrition data is transmitted to the client terminal 10 via the Internet 3 by the server-side data transmission/reception means 26 provided in the service provision server 20. The client terminal 10 displays the received evaluated nutritional data on the display 1 (step S08). The user can view the displayed nutrition data and easily and quickly check his/her own nutrition state.

### [Example 3]

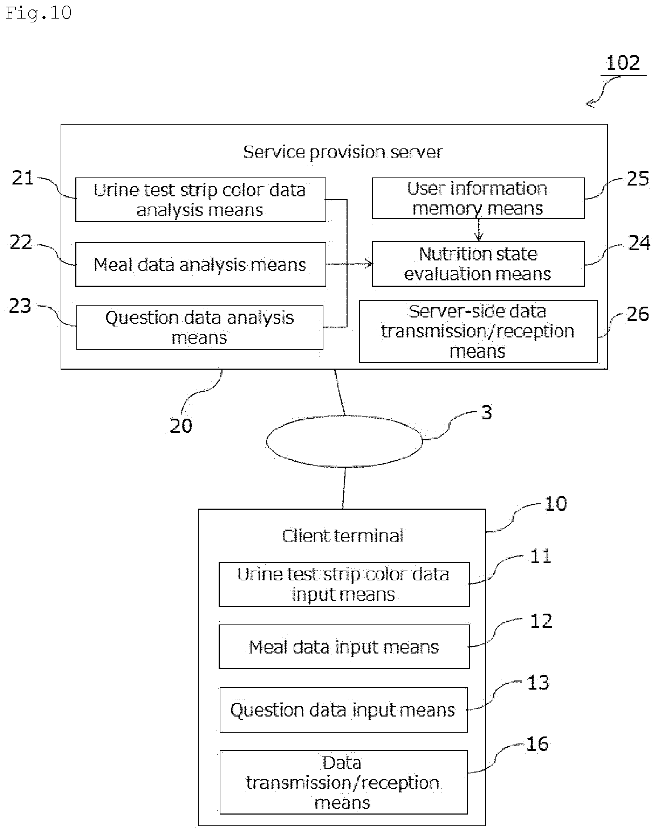

Fig. 10 shows a functional block diagram of the nutrition state analysis system of Example 3. As shown in Fig. 10, the nutrition state analysis system 100 is a system in which a service provision server 20 and client terminals 10 transmit and receive data via the Internet 3.

The client terminal 10 is provided with urine test strip color data input means 11, meal data input means 12, question data input means 13 and data transmission/reception means 16. The urine test strip color data input means 11 inputs the color data of the urine test strip soaked in the collected urine, and the meal data input means 12 inputs the meal data of the day on which the urine was collected. The question data input means 13 is for inputting question data regarding physical condition on the day the urine was collected. The data transmission/reception means 16 transmits the color data of the urine test strip, the meal data, and the question data that have been input to the service provision server 20, and receives evaluation data regarding the nutrition state from the service provision server 20.

The service provision server 20 is provided with urine test strip color data analysis means 21, meal data analysis means 22, question data analysis means 23, nutrition state evaluation means 24, user information memory means 25 and server-side data transmission/reception means 26. The urine test strip color data analysis means 21 analyzes the color data of urine test strips, and the meal data analysis means 22 analyzes meal data. The question data analysis means 23 analyzes question data, and the user information memory means 25 stores user information. The nutrition state evaluation means 24 evaluates the user's nutrition state based on the analysis results respectively analyzed by the urine test strip color data analysis means 21, the meal data analysis means 22 and the question data analysis means 23, and the user information stored in the user information memory means 25. Also, the server-side data transmission/reception means 26 receives urine test strip color data, meal data, and question data from the client terminal 10, and transmits evaluation data of the nutrition state evaluation means 24 to the client terminal 10.

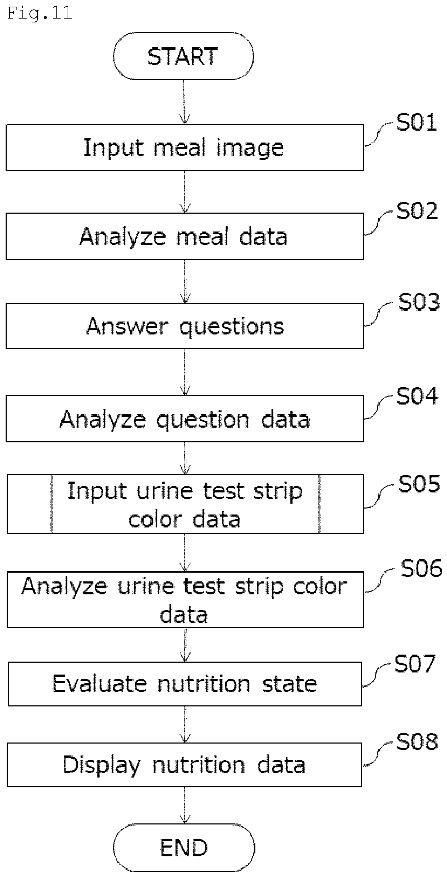

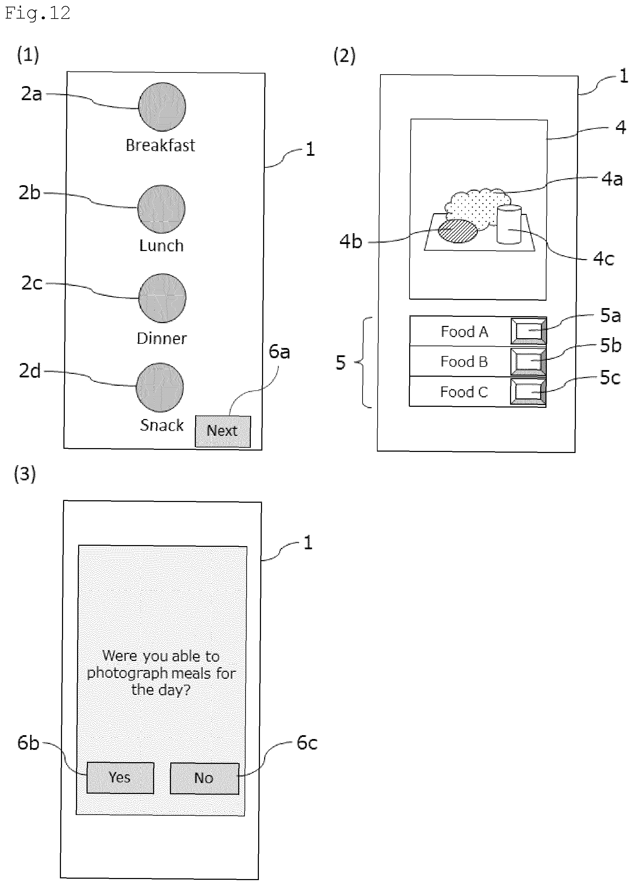

Next, the usage flow of the nutrition state analysis system of Example 3 will be described with reference to Figs. 11 and 12. Fig. 11 shows a schematic flow diagram of the nutrition state analysis system of Example 3.

As shown in Fig. 11, first, a meal image is input (step S01). Fig. 12 is a screen display image for inputting meal data, showing (1) a meal selection screen, (2) a food selection screen, and (3) a determination screen.

In Fig. 12(1), the display 1 of the client terminal 10 displays meal selection buttons (2a to 2d) and a button 6a. Specifically, a meal selection button 2a for inputting a breakfast image, a meal selection button 2b for inputting a lunch image, a meal selection button 2c for inputting a dinner image, and a meal selection button 2d for inputting a snack image are displayed. When the user selects one of the buttons (2a to 2d) and taps the button 6a labeled "Next", the screen transitions to an image registration screen (not shown), and a meal image is photographed on the image registration screen or an already photographed meal image is selected and registered.

When an image is photographed or registered, the meal image data is transmitted to the service provision server 20 via the Internet 3 by the data transmission/reception means 16 provided in the client terminal 10. The service provision server 20 performs image analysis on the received meal image data using the meal data analysis means 22, and presents food candidates included in the meal image to the user.

That is, when the user photographs an image or registers an image, the screen transitions to the food selection screen shown in FIG. 12(2). As shown in FIG. 12(2), an image 4 is registered in the display 1 as an example. The image 4 shows foods (4a-4c) on a tray. Under the image 4, a food candidate display field 5 is displayed. The food candidate display field 5 displays a list of analysis results of the food contained in the image 4. Although only the food name is displayed here, it is possible to display information other than the food name, such as quantity, weight, and estimated calorie content. In this Example, the image analysis in the meal data analysis means 22 uses a calomeal (registered trademark) API (Application Programming Interface) provided by Life Log Technology, inc.

The user compares the foods (4a to 4c) with the foods A to C displayed in the food candidate display field 5, and if they match, taps the food selection buttons (5a to 5c) arranged to the right of the food name. Also, although not shown here, when the matching food is not displayed in the food candidate display field 5, the user can also input the food name, amount, etc. in text form.

The food selection by the user is transmitted to the service provision server 20 via the Internet 3 by the data transmission/reception means 16 provided in the client terminal 10. This completes the registration of meal data for one meal. The service provision server 20 analyzes the received meal data using the meal data analysis means 22 (step S02).

When the selection of food for one meal is completed, the screen transitions to the determination screen shown in Fig. 12(3). If all the meal data for one day have been entered, tap the button 6b labeled "Yes", if not completed yet, tap the button 6c labeled "No".

After completing the input of meal data for one day, the user uses the data input means 13 provided in the client terminal 10 to answer the questions, as shown in Fig. 11 (step S03). The question data is transmitted to the service provision server 20 via the Internet 3 by the data transmission/reception means 16. The service provision server 20 analyzes the received question data using the question data analysis means 23 (step S04).

After completing the input of the question data, the user inputs urine test strip color data (step S05). The service provision server 20 analyzes the received urine test strip color data using the urine test strip color data analysis means 21 (step S06).

After analyzing the meal data, the question data, and the urine test strip color data, the service provision server 20 evaluates nutrition state using the nutrition state evaluation means 24 based on each analysis result and the user information stored in the user information memory means 25 (step S07).

After that, the evaluated nutrition data is transmitted to the client terminal 10 via the Internet 3 by the server-side data transmission/reception means 26 provided in the service provision server 20. The client terminal 10 displays the received evaluated nutrition data on the display 1 (step S08). The user can view the displayed nutrition data and easily and quickly check his/her own nutrition state.

In addition, regarding steps S03 to S08 shown in Fig. 11, descriptions of the same parts as in Example 2 are omitted.

### [Example 4]

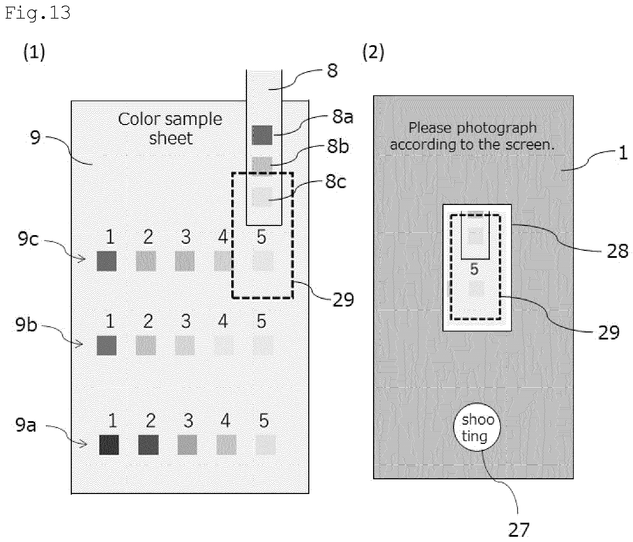

Fig. 13 is a screen display image for inputting urine test strip color data according to Example 4. Fig. 13(1) shows a color sample sheet image, and FIG. 13(2) shows a number photographing screen. In addition, except for the method of inputting urine test strip color data, the method is the same as that of Example 1.

In step S16 shown in Fig. 3, when the user is notified by the notification sound, the user compares the color sample sheet 9 and the test strip 8 shown in Fig. 13(1). On the color sample sheet 9, color samples (9a to 9c) corresponding to reaction sites (8a to 8c) are displayed. Therefore, the reaction sites (8a to 8c) and the color samples corresponding to numbers 1 to 5 in the color samples (9a to 9c) are compared, and the number corresponding to the most similar color is selected. Using the camera mounted on the client terminal 10, the selected number is included in the imaging range 28 shown in Fig. 13(2) so that it is photographed.

Specifically, the reaction site 8a is compared with the color sample corresponding to the numbers 1 to 5 in the color sample 9a, and the number considered to be most similar is included in the imaging range 28 shown in Fig. 13(2) and photographed. In FIG. 13(1), the color of the reaction site 8a is considered to be close to color number "2". Therefore, the shooting button 27 is tapped so that the number "2" is included in the imaging range 28, and the photographing is performed.

Next, the reaction site 8b is compared with the color sample corresponding to the numbers 1 to 5 in the color sample 9b, and the number that seems to be the most similar is photographed so that it is included in the imaging range 28 shown in Fig. 13(2). In Fig. 13(1), the color of the reaction site 8b is considered to be close to color number "2". Therefore, the shooting button 27 is tapped so that the number "2" is included in the imaging range 28, and the photographing is performed.

Finally, the reaction site 8c is compared with the color sample corresponding to the numbers 1 to 5 in the color sample 9c, and the number that seems to be the most similar is photographed so that it is included in the imaging range 28 shown in Fig. 13(2). In Fig. 13(1), the color of the reaction site 8c is considered to be close to color number "5". Therefore, the shooting button 27 is tapped so that the number "5" is included in the imaging range 28, and the photographing is performed.

In this Example, the order of imaging is described as the order of the reaction site 8a, the reaction site 8b, and the reaction site 8c, but the order is not limited to this. Also, the color number of which color sample should be imaged may be clearly indicated on the display 1 shown in Fig. 13(2).

In addition, for example, when photographing the reaction site 8c, as shown in the range 29, not only the number "5" but also at least one of a color of the reaction site 8c and the color corresponding to the number "5" in the color sample 9c may be photographed, so as to be included in the imaging range 28, and the color of the reaction site or the color of the color sample may be image-recognized and used for discrimination.

Alternatively, a method of inputting urine test strip color data by selecting a number or entering text instead of photographing the color number may be used.

### (Other examples)

1) The meal data input means 12 in Example 3 discriminates whether or not there is an input of meal data for the previous day before urine was collected in addition to the day urine is collected. Then, if there is no input of the meal data of the previous day, the user may be prompted to input the meal data of the previous day before urine was collected.
2) The order of answering the questions (step S03), analyzing the question data (step S04), inputting the urine test strip color data (step S05), and analyzing the urine test strip color data (step S06) shown in Fig. 8 are not limited to the order of Example 2, after inputting the urine test strip color data (step S05) and analyzing the urine test strip color data (step S06), answering the questions (step S03) and analyzing the question data (step S04) may be performed.
3) The order of inputting the meal images (step S01), analyzing the meal data (step S02), answering the questions (step S03), analyzing the question data (step S04), inputting the urine test strip color data (step S05), and analyzing the urine test strip color data (step S06) shown in Fig. 11 are not limited to the order of Example 3, and may be performed in any order. For example, after inputting urine test strip color data (step S05) and analyzing the urine test strip color data (step S06), answering the questions (step S03) and analyzing the question data (step S04) are performed, finally, inputting the meal images (step S01) and analyzing the meal data (step S02) may be performed.
4) The urine test strip color data analysis means 21 may be provided in the client terminal 10.

### [Industrial Applicability]

The present invention is useful for analysis of nutrition state using urine test strips.

### [Description of Symbols]

- 1: Display
- 2a ~ 2d: Meal selection button
- 3: Internet
- 4: Image
- 4a ~ 4c: Food
- 5: Food candidate display column
- 5a ~ 5c: Food selection button
- 6a ~ 6g: Button
- 7a ~ 7c: Symptom selection column
- 8: Urine test strip
- 8a ~ 8c: Reaction site
- 9, 90: Color sample sheet
- 9a ~ 9c, 91: Color sample
- 10: Client terminal
- 11: Urine test strip color data input means
- 12: Meal data input means
- 13: Question data input means
- 16: Data transmission/reception means
- 20: Service provision server
- 21: Urine test strip color data analysis means
- 22: Meal data analysis means
- 23: Question data analysis means
- 24: Nutrition state evaluation means
- 25: User information memory means
- 26: Server-side data transmission/reception means
- 27: Shoot button
- 28: Imaging range
- 29: Range
- 30a ~ 30d: Frame line
- 100 ~ 102: Nutrition state analysis system

## Claims

1. A nutritional state analysis system in which a client terminal and a service provision server transmit and receive data via a network, comprising:
the client terminal provided with a urine test strip color data input means for inputting the color data of the urine test strip soaked with collected urine, and a data transmission/reception means that transmits color data for an inputted urine test strip to the service provision server and receives evaluation data from the service provision server; and
the service provision server provided with a urine test strip color data analysis means for analyzing color data of urine test strip, a nutrition state evaluation means that evaluates the nutrition state of the user on the basis of the analysis result produced by the urine test strip color data analysis means, and a server-side data transmission/reception means that receives the color data for the urine test strip from the client terminal, and transmits evaluation data from the nutrition state evaluation means to the client terminal.

2. A nutritional state analysis system in which a client terminal and a service provision server transmit and receive data via a network, comprising:
the client terminal provided with a urine test strip color data input means for inputting the color data of the urine test strip soaked with collected urine, a question data input means for inputting question data regarding physical condition on the day urine was collected, and a data transmission/reception means that transmits color data for an inputted urine test strip and question data to the service provision server and receives evaluation data from the service provision server; and
the service provision server provided with a urine test strip color data analysis means for analyzing color data of the urine test strip, a question data analysis means for analyzing question data, a user information memory means for storing user information, a nutrition state evaluation means that evaluates the nutrition state of the user on the basis of the analysis result respectively analyzed by the urine test strip color data analysis means and the question data analysis means and the user information stored in the user information memory means, and a server-side data transmission/reception means that receives the color data for the urine test strip and the question data from the client terminal, and transmits evaluation data from the nutrition state evaluation means to the client terminal.

3. A nutritional state analysis system in which a client terminal and a service provision server transmit and receive data via a network, comprising:
the client terminal provided with a urine test strip color data input means for inputting the color data of the urine test strip soaked with collected urine, a meal data input means for entering meal data on the day urine was collected, a question data input means for inputting question data regarding physical condition on the day urine was collected, and a data transmission/reception means that transmits color data for an inputted urine test strip, meal data and question data to the service provision server and receives evaluation data from the service provision server; and
the service provision server provided with a urine test strip color data analysis means for analyzing color data of the urine test strip, a meal data analysis means for analyzing meal data, a question data analysis means for analyzing question data, a user information memory means for storing user information, a nutrition state evaluation means that evaluates the nutrition state of the user on the basis of the analysis result respectively analyzed by the urine test strip color data analysis means, the meal data analysis means and the question data analysis means and the user information stored in the user information memory means, and a server-side data transmission/reception means that receives the color data for the urine test strip, the meal data and the question data from the client terminal, and transmits evaluation data from the nutrition state evaluation means to the client terminal.

4. The nutrition state analysis system according to any one of claims 1 to 3,
wherein the urine test strip reacts with at least one of urinary calcium ions and urinary magnesium ions to develop a color, and the color density varies depending on the ion concentration, and
wherein the urine test strip color data analysis means analyzes the calcium or magnesium intake from the color data.

5. The nutrition state analysis system according to any one of claims 1 to 4,
wherein the urine test strip color data input means photographs the reaction site of the urine test strip colored by the collected urine and the reference color sample at the same time by the camera mounted on the client terminal, and
wherein the urine test strip color data analysis means analyzes the color code of the reaction site based on the color sample.

6. The nutrition state analysis system according to any one of claims 1 to 4, wherein the urine test strip color data input means acquires color information indicated by a color sample that approximates the color of the urine test strip colored by the collected urine.

7. The nutrition state analysis system according to claim 6, wherein the urine test strip color data input means prompts the user to photograph the color information indicated in the color sample by the camera means mounted on the client terminal.

8. The nutrition state analysis system according to claim 3, wherein the meal data input means discriminates whether or not there is an entry of meal data for the day before the urine was collected in addition to the day the urine was collected, and prompts the user to enter the meal data for the day before urine collection in the case in which the meal data of the previous day has not been entered.
